# EUROPEAN PATENT APPLICATION

(11) **EP 4 745 127 A1**
(43) Date of publication of application: **20.05.2026**
(21) Application number: 24838697.1
(22) Date of filing: 05.07.2024
(51) Int. Cl.: C07D 239/47, C07D 239/48, C07D 401/14, A61K 31/506, A61P 17/02, A61P 25/16, A61P 35/00, A61P 35/02, A61P 35/04, A61P 3/04, A61P 3/10, A61P 25/28, A61P 13/12, A61P 11/00, A61P 9/00, A61P 17/00, A61P 25/00, A61P 3/00

(54) **PYRIMIDINAMINE NUAK INHIBITOR AS WELL AS PREPARATION METHOD THEREFOR AND USE THEREOF**

(30) Priority: 13.07.2023 CN 202310866091
(71) Applicant: Technoderma Medicines Inc., Chengdu, Sichuan 610219 (CN)
(72) Inventor: FANG, Wenkui Ken, California 92618 (US); LI, Guanqun, Oregon 97239 (US); CAI, Yuting, Chengdu, Sichuan 610219 (CN); WANG, Zengquan, Nevada 89113 (US)
(74) Representative: Manitz Finsterwald Patent- und Rechtsanwaltspartnerschaft mbB
(86) International application number: PCT/CN2024/103750
(87) International publication number: WO 2025/011444

(57) **Abstract**

Provided in the present application are pyrimidinamine compounds, characterized in that the pyrimidinamine compounds are compounds represented by formula I, or stereoisomers, tautomers, isotope derivatives, hydrates, solvates, prodrugs and pharmaceutically acceptable salts thereof. The pyrimidinamine compounds of the present application have excellent NUAK1/NUAK2 kinase inhibitory activity, and thus can be used for preventing or treating the following diseases by means of inhibiting NUAK1 or NUAK2: neuropsychiatric diseases, metabolic diseases, tumors, visceral fibrosis diseases, and skin fibrosis diseases.

## Description

### CROSS-REFERENCE TO RELATED APPLICATIONS

The present application claims priority to Chinese Patent Application No. 202310866091.6, filed on July 13, 2023, which is incorporated herein by reference in its entirety..

### TECHNICAL FIELD

The present disclosure relates to the field of small molecule compounds, specifically to a pyrimidinamine-based NUAK inhibitor and a preparation method and use thereof. The compound, by inhibiting NUAK1 or NUAK2, is useful for the prevention or treatment of the following diseases: neuropsychiatric diseases, metabolic diseases, tumors, visceral fibrotic diseases, and skin fibrotic diseases, or used solely for alleviating traumatic and postoperative scars.

### BACKGROUND

Protein kinases are a group of important functional proteins participating in the regulation of metabolism, polarity, growth, division, and differentiation of cells, and the human genome encodes more than 500 types of protein kinases that can transfer phosphate groups from ATP (adenosine triphosphate) to specific serine, threonine, or tyrosine residues of substrate proteins for phosphorylation. Most protein kinases are serine/threonine kinases, and there are less than 100 types of tyrosine kinases. Adenosine monophosphate-activated protein kinase (AMPK) belongs to serine/threonine kinases (STK) and is an important regulator of cellular energy homeostasis in mammals. AMPK can regulate glucose and lipid metabolism, cell proliferation, and cell polarity by sensing a change in an intracellular AMP (Adenosine monophosphate)/ATP or ADP (adenosine diphosphate)/ATP ratio under metabolic stress (for example, hypoxia or heat shock), and therefore, can be referred to as a metabolic sensor protein. AMPK is evolutionarily highly conserved and a heterotrimeric protein consisting of an α subunit (containing a kinase domain, KD), a β subunit (regulating kinase activity), and a γ subunit. The γ subunit contains four cystathionine-β-synthase (CBS) domains and is responsible for detecting changes in the intracellular AMP/ATP and ADP/ATP ratios (Hardie DG, Trends in Cell Biology. 2016, 26:190).

AMPK dysfunction can cause a variety of diseases such as obesity, diabetes, inflammatory diseases and tumors, and therefore, regulation of AMPK function by the body is critical. First of all, phosphorylation of threonine 172 (T172) on the α subunit by upstream kinases is required for AMPK activation. Three types of upstream kinases of AMPK have been found by now, namely, liver kinase B1 (LKB1), Ca²⁺/calmodulin-dependent PK kinase 2 (CaMKK2) and transforming growth factor-β-activated kinase 1 (TAK1). Corresponding to the activation of AMPK via phosphorylation of the T172 site by the upstream kinase, three protein phosphatases are found to inhibit the activity of AMPK by removing a phosphate group from T172, including protein phosphatase 2A (PP2A), protein phosphatase 2C (PP2C) and Mg²⁺/Mn²⁺-dependent protein phosphatase 1E (PPM1E). When cells are in a low-energy state (high AMP/ATP ratio or ADP/ATP ratio), the α subunit KD and γ subunit of AMPK are tightly cross-linked, while the β subunit is myristoylated, preventing protein phosphatases from accessing the T172 site and thus maintaining AMPK activation. In addition, when cells are in a high-energy state, KD and γ subunit are detached, and T172 is exposed to phosphatase, thereby causing inactivation of AMPK (Steinberg GR, Nature Reviews Drug Discovery. 2019, 18:527).

In addition to AMPK upstream kinases and protein phosphatases that can activate and inactivate AMPK respectively, there are also a type of AMPK-related kinases (ARK) participating in the regulation of AMPK functions, and a total of 12 ARKs (BRSK1, BRSK2, NUAK1, NUAK2, QIK, QSK, SIK, MARK1, MARK2, MARK3, MARK4 and MELK) have been found by now and all belong to serine/threonine kinases. Kinase domains of ARKs have high homology with the α subunit of AMPK. All ARKs can be activated by LKB1 except MELK, their kinase-activating phosphorylation sites are equivalent to AMPK T172, and ARKs also functionally participate in the regulation of metabolism, proliferation and polarity of cells. However, unlike AMPK with regulatory subunits, ARKs cannot be directly regulated by the intracellular AMP/ATP ratio (Bright NJ, Acta Physiologica. 2009, 196:15).

ARKs are classified into several ARK subfamilies by different protein structures and functional characteristics. NUAK subfamily (Nu (novel) and AMPK-related kinase) of ARKs includes two members of NUAK1 (initially named ARK5) and NUAK2 (initially named sucrose nonfermenting-like/AMPK related kinase, SNARK). Amino acid sequences of the two members are approximately 55% homologous, and molecular weights of NUAK1 and NUAK2 estimated according to amino acid sequences are 76 kDa and 69 kDa respectively. They share highly similar protein structures, with an N-terminal kinase domain and a C-terminal ubiquitin-binding domain, and currently, it is yet unclear whether NUAK is a heterotrimeric structure like AMPKs. NUAK1 and NUAK2 are expressed in most tissues, an expression level of NUAK1 in organs and tissues such as brain, skin, muscle, upper gastrointestinal tract and endocrine is significantly higher than that in other parts, organs and tissues with the highest expression level of NUAK2 are the gastrointestinal tract, female reproductive system, skin, bone, brain, endocrine and the like, and the expression of NUAK2 shows more tissue specificity. It should be noted that NUAK1 and other ARKs and AMPK proteins are mainly distributed in cytoplasm, while NUAK2 is mainly distributed in nucleus, and there are indications that NUAK2 participates in the expression of genes related to metabolic stress as a transcriptional regulator (Sun X, J of Molecular Endocrinology. 2013, 51:R15).

As serine/threonine kinases, NUAK1 and NUAK2 can phosphorylate a variety of protein substrates, including proteins participating in cell signaling and metabolism, cell proliferation, cell apoptosis and autophagy, and cytoskeletal tissues. As it is known, NUAK1 can phosphorylate AMPK, LATS1/2, p53 tumor suppressor protein, and myosin phosphatase target subunit 1 (Mypt1) regulating actin cytoskeletal tissue. NUAK2 can phosphorylate transcription factors Gli3 and FoxO1 of Hedgehog signaling pathway, kinesin light chain 1 (KLC1), Rho GDP dissociation inhibitor α (Rho GDIα), and the like.

The functional regulation of NUAK1 and NUAK2 relates to a variety of intracellular signaling systems. In addition to being activated via phosphorylation by LKB1 (T211, equivalent to T172 of AMPK) and activated by Ca²⁺/PKC, NUAK1 is the only member of the AMPK-related protein family that can be activated by Akt, and an increase in the activity of NUAK1 is also found in the activation of signaling pathways of some growth factors such as insulin-like growth factor 1 (IGF1). Contraction states of skeletal muscle cells are also accompanied by increased activity of NUAK1. Both NUAK1 and NUAK2 can be activated by LKB1 (T211/NUAK1, T208/NUAK2, equivalent to T172 of AMPK), but NUAK2 can be autophosphorylated, and NUAK2 can interact with ubiquitin-specific protease 9, X-linked (USP9X), which is a deubiquitinase and can maintain activity of NUAK2. Different intracellular stimuli such as low osmotic pressure, DNA damage, oxidation and nutrient deficiencies can all lead to the activation of NUAK2, and the activation of NUAK2 is also found in the contraction of skeletal muscle cells (Brooks D, Data in Brief. 2022, 43:108482).

A number of studies have revealed that NUAK plays an important role in pathogenesis of diseases such as metabolic diseases, tumors, neurodegenerative diseases and fibrotic diseases, homozygous NUAK1/NUAK2 knockout mice basically die in the embryonic period, mice with heterozygous NUAK1 gene deleted exhibit systemic and nervous tissue hypoplasia (for example, cerebral cortex and peripheral neurons), heterozygous gene mutation of human NUAK1 is associated with autism spectrum disorders (ASD), cognitive deficits, attention deficit/hyperactivity disorder (AD/HD) and schizophrenia. Deletion of the human NUAK2 gene causes anencephaly, which is a severe neural tube malformation that causes defective fetal development and whose pathogenesis is related to loss of YAP function (Bonnard C, J Exp Med. 2020, 217:e20191561). Skeletal muscle cell-specific knockout of the NUAK1 gene can avoid high-fat diet-induced subclinical diabetes. However, a phenotype of mice with heterozygous NUAK2 gene deleted is similar to a series of clinical manifestations of human type II diabetes mellitus with obesity, and these phenomena may be related to imbalance of cellular autophagy mechanism (Bennison SA, Cellular Signaling. 2022, 100:110472; Blazejewski SM, Scientific Reports. 2011, 11:8156).

Activation of NUAK1 caused by Akt and other protein kinases can promote survival of tumor cells in an environment with energy deficiency and protect tumor cells from apoptosis, and NUAK2 also inhibits, through a similar mechanism, apoptosis induced by TNF α and CD95. In addition, after activation, NUAK1 promotes invasion and metastasis of tumor cells by upregulating the matrix metalloproteinases (MMP), while NUAK2 participates in occurrence of tumor metastasis by enhancing tumor cell motility (Hou X, Oncogen. 201, 30:2933; Chen Y, Cell Death and Disease. 2020, 11:712; Molina E, Cells. 10:2760; Humbert N, The EMBO Journal. 2010, 29:376). A lot of evidence reveals that NUAK1 and NUAK2 play a role in tumor formation and metastasis, NUAK2 has a stronger function in promoting tumor formation and metastasis than NUAK1, and NUAK2 inhibitors should be more likely to become new targeted antitumor drugs than NUAK1 inhibitors.

Recent studies have revealed that NUAK plays a critical role in development of tissue fibrosis through interaction with transforming growth factor-β (TGF-β) signaling pathway. First, TGF-β can upregulate gene transcription of NUAK1 and NUAK2 in a variety of epithelial cells such as keratinocytes and human dermal fibroblasts, while inactivation of MAPK (ERK1/2 and p38) signals inhibits expression of TGF-β-dependent NUAK2. In addition, NUAK2 inhibits degradation of SMAD3 in cells by binding to the structurally crosslinking domain and MH2 domain of the intracellular signaling mediator SMAD3 protein of TGF-β. A similar mechanism exists between NUAK2 and TβRI. TGF-β-induced expression of genes encoding profibrotic molecules such as fibronectin (FN), plasminogen activator inhibitor 1 (PAI1) and tissue inhibitor of metalloproteinase-1 (TIMP1) depends on the presence of NUAK2. These findings indicate that NUAK2 promotes fibrogenesis. Studies have also revealed that NUAK1 promotes fibrosis of kidney, lung and liver by upregulating two signaling pathways of TGF-β and YAP, and in animal experiments, inhibition of NUAK1 can alleviate scars and mature scar tissues caused by new trauma (Gill MK, Nature Communications. 2018. 9:3510). Interestingly, studies have revealed that when expression of genes encoding FN in keratinocytes with NUAK1 gene knocked out is upregulated, NUAK1 may inhibit fibrosis by influencing TGF-β signaling through a negative feedback mechanism (van de Vis RAJ, Cancers. 2021, 13:3377). In general, inhibition of NUAK may effectively inhibit fibrosis processes of tissues and organs involved.

Given their structural and functional properties, kinases have long been ideal targets for small-molecule drugs, and NUAK1 and NUAK2 are involved in the occurrence of a variety of diseases, some NUAK inhibitors have been in an early stage of development by now, but their efficacy and safety are yet undetermined (Banerjee S, The Biochemical Journal. 2014, 457:215). Therefore, development of selective NUAK inhibitors and dual-target NUAK1/2 inhibitors is expected to provide a new direction for innovative treatments of neuropsychiatric diseases (for example, Parkinson's disease and Alzheimer's disease), metabolic diseases (for example, diabetes, hyperlipidemia and obesity), tumors (for example, liver cancer, leukemia, lymphoma and tumor metastasis), visceral fibrotic diseases (for example, liver cirrhosis, renal fibrosis, pulmonary fibrosis and post-myocarditis sequelae), and skin fibrotic diseases (for example, scleroderma, keloids and hypertrophic scars), or solely for alleviating traumatic and postoperative scars. Developing next-generation NUAK inhibitors has significant potential clinical application value.

### SUMMARY OF THE INVENTION

An objective of the present disclosure is to obtain an effective NUAK1/NUAK2 inhibitor that is useful for preparing medicaments for prevention or treatment of the following diseases: neuropsychiatric diseases (for example, Parkinson's disease and Alzheimer's disease), metabolic diseases (for example, diabetes, hyperlipidemia and obesity), tumors (for example, liver cancer, leukemia, lymphoma and tumor metastasis), visceral fibrotic diseases (for example, liver cirrhosis, renal fibrosis, pulmonary fibrosis and post-myocarditis sequelae), and skin fibrotic diseases (for example, scleroderma, keloids and hypertrophic scars), or solely for alleviating traumatic and postoperative scars.

To achieve the foregoing objective, an aspect of the present disclosure provides a pyrimidinamine-based compound, where the pyrimidinamine-based compound is a compound represented by Formula I shown below, or a stereoisomer, a geometric isomer, a tautomer, an isotopic derivative, a hydrate, a solvate, a prodrug, or a pharmaceutically acceptable salt thereof, 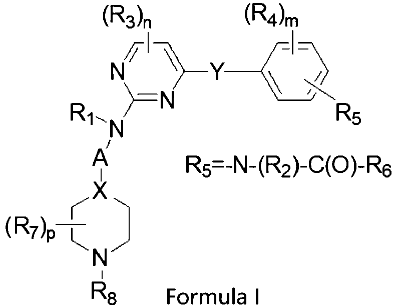
where A is 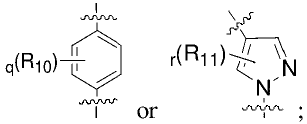
X is N or CH; Y is O or NR₉,
R₁, R₂, R₈, and R₉ are each independently H or an optionally substituted C1-C8 alkyl group;
R₃, R₄, R₇, R₁₀, and R₁₁ are each independently halogen, an amino group, a hydroxyl group, a nitro group, a cyano group, a sulfhydryl group, an optionally substituted C1-C8 alkyl group, an optionally substituted C1-C8 alkoxy group, an optionally substituted C1-C8 alkylthio group, or an optionally substituted C1-C8 alkylamino group;
R₆ is an optionally substituted 3- to 10-membered cycloalkyl group or an optionally substituted 3- to 10-membered heterocycloalkyl group, where the substituent on the cycloalkyl group or heterocycloalkyl group is one or more selected from the group consisting of halogen, an amino group, a hydroxyl group, a nitro group, a cyano group, a sulfhydryl group, an optionally substituted C1-C8 alkyl group, an optionally substituted C1-C8 alkoxy group, an optionally substituted C1-C8 alkylthio group, and an optionally substituted C1-C8 alkylamino group;
the substituent on the C1-C8 alkyl group is one or more selected from the group consisting of halogen, an amino group, a hydroxyl group, a nitro group, a cyano group, and a sulfhydryl group; and
n and r are each 0, 1, or 2; and m, p, and q are each 0, 1, 2, 3, or 4.

In one set of embodiments, Y is O or NH.

In one set of embodiments, R₆ is an optionally substituted 3- to 10-membered cycloalkyl group, preferably an optionally substituted 3- to 6-membered cycloalkyl group, more preferably a 3- to 6-membered cycloalkyl group substituted with halogen, and even more preferably, R₆ is a 3- to 6-membered cycloalkyl group substituted with fluorine.

In one set of embodiments, R₆ is

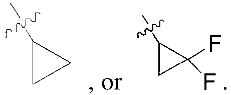

In one set of embodiments, R₅ is 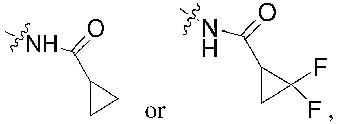 preferably, R₅ is

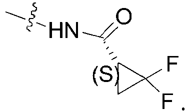

In one set of embodiments, R₅ is substituted at the ortho- or meta-position to Y.

In one set of embodiments, the compound represented by Formula I is represented by Formula I-1:

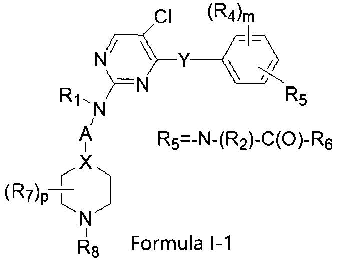

In one set of embodiments, X is N, and R₈ is H or a methyl group.

In one set of embodiments, the compound represented by Formula I is represented by Formula I-2 or Formula I-3:

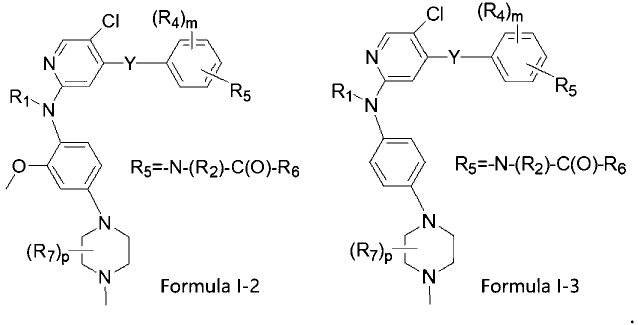

In one set of embodiments, the compound represented by Formula I is one of the following compounds:

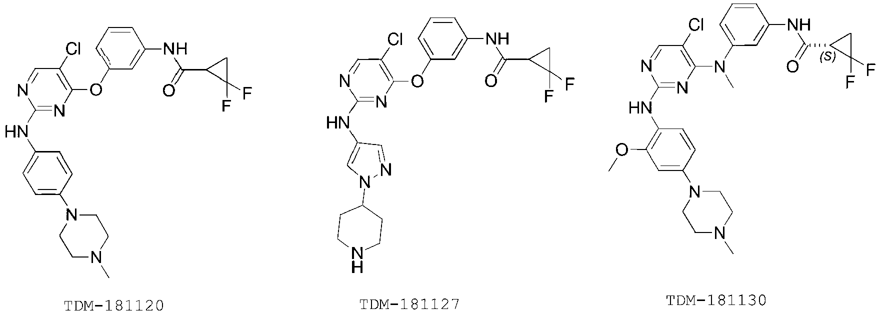

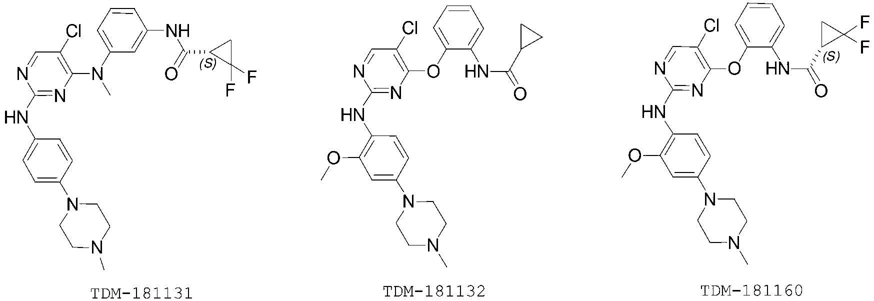

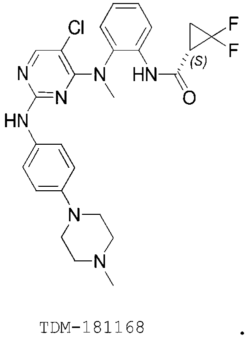

In one set of embodiments, the pharmaceutically acceptable salt is a formate salt.

In one set of embodiments, the isotopic derivative is a deuterated compound.

Another aspect of the present application further provides a method for preparing the pyrimidinamine-based compound, including: preparing the compound of Formula I from a compound of Formula II and a compound of Formula III; or
preparing a compound of Formula V from a compound of Formula IV and a compound of Formula II, and then preparing the compound of Formula I from the compound of Formula V;
optionally, the preparation process includes necessary protection and deprotection steps; 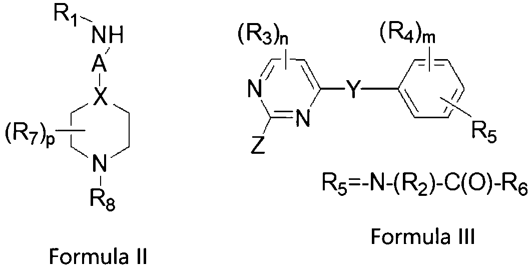 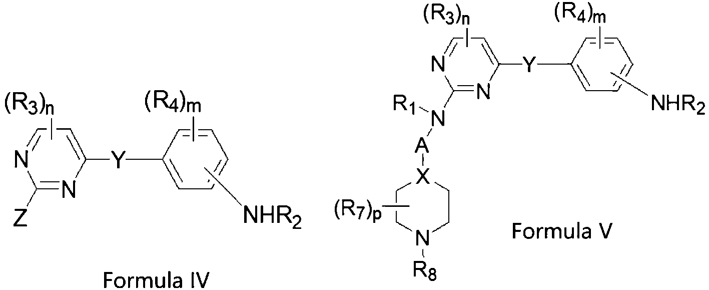
where Z is a leaving group, preferably halogen, more preferably Cl, and other groups are as defined above.

In one set of embodiments, the amino or imino group on the six-membered nitrogen-containing heterocycle in Formula II is first protected, the protected compound is then coupled with a compound of Formula III, followed by removing the protecting group to obtain the compound of Formula I.

In one set of embodiments, the amino or imino group attached to R₂ in the compound of Formula IV is first protected, and the amino group or the imino group on the six-membered nitrogen-containing heterocycle in Formula II is optionally protected. The protected compound of Formula IV is then coupled with the optionally protected compound of Formula II, followed by removing the protecting group on the amino or imino group attached to R₂ to obtain the compound of Formula V. The compound of Formula V is subsequently reacted with R₆-COOH, and the protecting group on ring B is optionally removed to obtain the compound of Formula I.

The present application provides a pharmaceutical composition, in which the pyrimidinamine-based compound according to the present application is used as an active ingredient, and the pharmaceutical composition further contains a pharmaceutically acceptable carrier or excipient.

The present application further provides the use of the pyrimidinamine-based compound in the preparation of a NUAK1 or NUAK2 inhibitor.

The present application further provides the use of the pyrimidinamine-based compound in preparation of a medicament, wherein the compound is useful for the prevention or treatment of the following diseases by inhibiting NUAK1 or NUAK2: neuropsychiatric diseases, metabolic diseases, tumors, visceral fibrotic diseases, or skin fibrotic diseases.

In one set of embodiments, the disease is Parkinson's disease, Alzheimer's disease, diabetes, hyperlipidemia, obesity, liver cancer, leukemia, lymphoma, tumor metastasis, liver cirrhosis, renal fibrosis, pulmonary fibrosis, post-myocarditis sequelae, scleroderma, keloids, or hypertrophic scar; or the compound is used solely for alleviating a traumatic or postoperative scar.

### Beneficial effects of the present disclosure:

The present disclosure provides a class of pyrimidinamine-based compounds, and *in vitro* kinase activity inhibition assays show that the compounds according to the present disclosure have excellent inhibitory activity against NUAK1 or NUAK2 kinases. The compounds are useful for the prevention or treatment of the following diseases by inhibiting NUAK1 or NUAK2: neuropsychiatric diseases, metabolic diseases, tumors, visceral fibrotic diseases, or skin fibrotic diseases.

### DETAILED DESCRIPTION

The present disclosure is described in detail hereinafter. It should be understood that the specific embodiments described herein are intended merely to illustrate and explain the present disclosure, and are not intended to limit the present disclosure.

The endpoints and any values of the ranges disclosed herein are not limited to those precise ranges or values, and such ranges or values should be understood to include values approximating those ranges or values. For numerical ranges, combinations may be made between the endpoint values of each range, between the endpoint values of each range and individual point values, and between individual point values to obtain one or more new numerical ranges, and such numerical ranges are to be regarded as specifically disclosed herein.

Before describing the present disclosure in detail, it should be understood that the terms used herein are for the purpose of describing particular embodiments only and are not intended to limit the scope of the present disclosure, which is to be limited solely by the appended claims. For a more complete understanding of the present disclosure described herein, the following terms are employed, with definitions set forth below. Unless otherwise defined, all technical and scientific terms used herein have the same meaning as commonly understood by one of ordinary skill in the art to which this disclosure belongs.

### Definitions

Unless otherwise specified, the following terms as used in the present disclosure have the definitions set forth below.

Features illustrated or described as part of one embodiment or group of embodiments may be used in another embodiment or group of embodiments to produce further embodiments.

In the present disclosure, 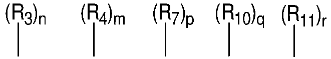 on a cyclic moiety indicates that n R₃, m R₄, p R₇, q R₁₀, or r R₁₁ may be attached at any possible position on the cyclic moiety.

In the present disclosure, 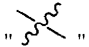 in some substituents represents the point of attachment. For moiety A, the upper position is attached to the amino group, and the lower position is attached to the six-membered nitrogen-containing heterocycle.

Unless otherwise specified, the term "optionally substituted" means that the hydrogen atoms on the substituted group are unsubstituted, or one or more substitutable positions on the substituted group are each independently substituted with substituent(s) each independently selected from the group consisting of deuterium, halogen, an amino group, a hydroxyl group, a nitro group, a cyano group, a sulfhydryl group, oxo, an optionally substituted C1-C8 alkyl group, an optionally substituted C1-C8 alkoxy group, an optionally substituted C1-C8 alkylthio group, and an optionally substituted C1-C8 alkylamino group. The substituent on the C1-C8 alkyl group is one or more selected from the group consisting of deuterium, halogen, an amino group, a hydroxyl group, a nitro group, a cyano group, and a sulfhydryl group. When the substituent is "oxo", it means that two hydrogen atoms at the same substitution position are replaced by an oxygen atom.

The term "cycloalkyl group" refers to a saturated monocyclic, bicyclic, or tricyclic hydrocarbon system containing from 3 to 10 carbon atoms, wherein the monocyclic, bicyclic, or tricyclic system contains no aromatic ring. Bicyclic groups include bridged rings, spiro rings, fused rings, and the like. Preferably, the cycloalkyl group contains from 3 to 10 carbon atoms (C3-10 cycloalkyl), more preferably from 3 to 8 carbon atoms (C3-8 cycloalkyl), from 3 to 6 carbon atoms (C3-6 cycloalkyl), from 4 to 6 carbon atoms (C4-6 cycloalkyl), or from 5 to 6 carbon atoms (C5-6 cycloalkyl). Examples include, but are not limited to, a cyclopropyl group, a cyclobutyl group, a cyclopentyl group, a cyclohexyl group, a methylcyclopropyl group, a 2-ethyl-cyclopentyl group, and a dimethylcyclobutyl group.

The term "heterocycloalkyl group" refers to a saturated monocyclic, bicyclic, or polycyclic hydrocarbon group, preferably having from 3 to 10 ring atoms, where one, two, three or more ring atoms are selected from N, O, and S, and the remaining ring atoms are carbon atoms, including bridged rings, spiro rings, fused rings, and the like. Preferably, the heterocycloalkyl group has from 3 to 8 ring atoms (3- to 8-membered heterocycloalkyl), from 3 to 6 ring atoms (3- to 6-membered heterocycloalkyl), from 4 to 6 ring atoms (4- to 6-membered heterocycloalkyl), or from 5 to 6 ring atoms (5- to 6-membered heterocycloalkyl). The number of heteroatoms is preferably from 1 to 4, more preferably from 1 to 3 (i.e., 1, 2, or 3). The heterocycloalkyl may be a 5- to 6-membered monocyclic heterocycloalkyl containing 1 or 2 N atoms. Examples of heterocycloalkyl groups include a pyrrolidinyl group, an imidazolidinyl group, a tetrahydrofuranyl group, a piperidinyl group, a piperazinyl group, a pyranyl group, an aziridinyl group, an oxiranyl group, a thiiranyl group, an azetidinyl group, an oxetanyl group, a thietanyl group, oxacyclohexyl, a morpholinyl group, a thiomorpholinyl group, a dioxanyl group, a dithianyl group, an oxazolidinyl group, a thiazolidinyl group, a pyrazolidinyl group, an imidazolinidinyl group, and the like.

The term "alkyl group" refers to a monovalent saturated aliphatic hydrocarbon group, preferably a straight or branched chain group containing from 1 to 8 carbon atoms (C1-8 alkyl; the number of carbon atoms is 1, 2, 3, 4, 5, 6, 7, or 8), more preferably from 1 to 6 carbon atoms (C1-6 alkyl; the number of carbon atoms is 1, 2, 3, 4, 5, or 6). Examples include, but are not limited to, a methyl group, an ethyl group, an n-propyl group, an isopropyl group, an n-butyl group, an isobutyl group, a tert-butyl group, a sec-butyl group, an n-pentyl group, a neopentyl group, a 1,1-dimethylpropyl group, a 1,2-dimethylpropyl group, a 2,2-dimethylpropyl group, a 1-ethylpropyl group, a 2-methylbutyl group, a 3-methylbutyl group, an n-hexyl group, an n-heptyl group, and an n-octyl group.

The terms "alkoxy group", "alkylthio group", and "alkylamino group" refer to -O-alkyl group, -S-alkyl group, -NH-alkyl group or dialkylamino group, respectively, where the alkyl group is as defined above. Representative examples include, but are not limited to, a methoxy group, an ethoxy group, a propoxy group, an isopropoxy group, a butoxy group, a 1-methylpropoxy group, a 2-methylpropoxy group, a tert-butoxy group, a methylthio group, an ethylthio group, a propylthio group, an isopropylthio group, a butylthio group, a 1-methylpropylthio group, a 2-methylpropylthio group, a tert-butylthio group, a methylamino group, an ethylamino group, a propylamino group, a dimethylamino group, a diethylamino group, a dipropylamino group, and a methylethylamino group.

The term "halogen" refers to F, Cl, Br, and I.

The active compound of the present disclosure is understood to include the compound itself, as well as its stereoisomers, tautomers, isotopic derivatives, hydrates, solvates, prodrugs, or pharmaceutically acceptable salts thereof. The stereoisomers, tautomers, hydrates, solvates, prodrugs, and pharmaceutically acceptable salts of the compound may be prepared by conventional techniques in the art, and exert the same or similar effects *in vitro* and *in vivo* through substantially the same mechanism of action as said compound.

The term "stereoisomer" refers to isomers arising from the different spatial arrangement of atoms in a molecule, including configurational isomers and conformational isomers. Configurational isomers further include geometric isomers (or cis-trans isomers) and optical isomers (including enantiomers and diastereomers). Geometric isomers may exist in the present compounds. Optical isomers are substances having identical molecular structures, similar physicochemical properties, but different optical activities. The compounds according to the present disclosure may contain asymmetrically substituted carbon atoms in either the R or S configuration, wherein the terms "R" and "S" are as defined in IUPAC 1974 Recommendations for Section E, Fundamental Stereochemistry, Pure Appl. Chem. (1976) 45, 13-10. Compounds containing asymmetrically substituted carbon atoms (with equal amounts of R and S configurations) are racemic at those carbon atoms. An excess of one configuration relative to the other results in a higher proportion of that configuration, preferably an excess of about 85% to 90%, more preferably an excess of about 9% to 99%, even more preferably an excess of greater than about 99%. Accordingly, the present disclosure includes racemic mixtures, individual relative and absolute optical isomers, and mixtures of relative and absolute optical isomers.

The term "tautomer" refers to structural isomers having different energies that are interconvertible via a low energy barrier. Where tautomerization is possible (e.g., in solution), a chemical equilibrium of tautomers can be reached. For example, proton tautomers (also called prototropic tautomers) include interconversions via migration of a proton, such as keto-enol tautomerization and imine-enamine tautomerization. Valence tautomers include interconversions via reorganization of some bonding electrons.

The term "isotopic derivative" means that the compound of the present disclosure may exist in an isotopically labeled or enriched form, containing one or more atoms whose atomic weight or mass number differs from the atomic weight or mass number of the most abundant naturally occurring atom. Isotopes may be radioactive or non-radioactive. Isotopes of hydrogen, carbon, phosphorus, sulfur, fluorine, chlorine, and iodine include, but are not limited to, ²H, ³H, ¹³C, ¹⁴C, ¹⁵N, ¹⁸O, ³²P, ³⁵S, ¹⁸F, ³⁶Cl, and ¹²⁵I. Compounds containing other isotopes of these and/or other atoms are within the scope of the present disclosure. The isotopically labeled compounds of the present disclosure may be prepared by general methods known to those of ordinary skill in the art.

The term "hydrate" refers to an association formed by one or more water molecules and the compound of the present disclosure.

The term "solvate" refers to an association formed by one or more solvent molecules and the compound of the present disclosure.

The term "prodrug" refers to a derivative of an active drug designed to improve certain identified undesirable physical or biological properties. Physical properties typically relate to solubility (excessively high or insufficient lipid or aqueous solubility) or stability, while problematic biological properties include overly rapid metabolism or poor bioavailability, which may themselves be related to physicochemical properties.

The term "pharmaceutically acceptable salt" refers to a salt that is, within the scope of sound medical judgment, suitable for use in contact with the tissues of mammals, especially humans, without undue toxicity, irritation, allergic response, and the like, and commensurate with a reasonable benefit/risk ratio. When the compound is basic, pharmaceutically acceptable salts include salts formed from inorganic acids and salts formed from organic acids. When the compound is acidic, pharmaceutically acceptable salts include salts formed from inorganic bases and/or organic bases.

The term "pharmaceutically acceptable carrier" or "pharmaceutically acceptable excipient" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent, emulsifier, or the like that is approved by a relevant governmental regulatory authority for use in humans or livestock.

As used herein, the term "treatment" refers to any administration of a therapeutic agent according to a therapeutic regimen that achieves a desired effect, i.e., partial or complete reduction, amelioration, alleviation, inhibition, delay of onset, reduction in severity, and/or reduction in the incidence of one or more symptoms or features of a particular disease, disorder, and/or condition. In some embodiments, administration of a therapeutic agent according to a therapeutic regimen is associated with the achievement of the desired effect. Such treatment may be administered to a subject who does not exhibit signs of the relevant disease, disorder, and/or condition and/or to a subject who exhibits only early signs of the disease, disorder, and/or condition. Alternatively or additionally, such treatment may be administered to a subject who exhibits one or more established signs of the relevant disease, disorder, and/or condition. In some embodiments, treatment may be administered to a subject diagnosed with the relevant disease, disorder, and/or condition. In some embodiments, treatment may be administered to a subject known to have one or more predisposing factors that are statistically associated with an increased risk of developing the relevant disease, disorder, and/or condition.

According to the present disclosure, the medicament prepared for the pharmaceutical use described herein may contain, in addition to the pyrimidinamine-based compound of the present disclosure as an active ingredient, one or more other agents useful for the prevention or treatment of the relevant diseases as a further active ingredient. When the medicament contains multiple active ingredients, the active ingredients may be administered simultaneously, sequentially, or separately at the discretion of a physician.

In the following, the effects of specific compounds of the present disclosure are described in detail with reference to examples.

### Examples

### Example 1 General Method for Synthesizing Compound 518 (TDM-181118)

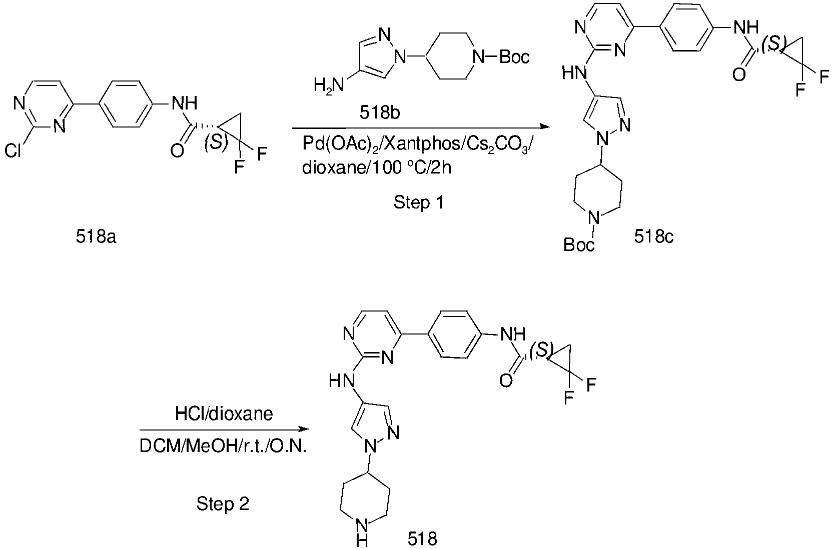

### Step 1: Compound 518c

### Tert-butyl (S)-4-(4-((4-(4-(2,2-difluorocyclopropane-1-carboxamido)phenyl)pyrimidin-2-yl)amino)-1H-pyrazol-1-yl)piperidine-1-carboxylate

1,4-dioxane (2 mL) was added to a mixture of compound 518a (20 mg, 0.065 mmol), compound 518b (26 mg, 0.097 mmol), palladium acetate, xantphos (37 mg, 0.065 mmol) and cesium carbonate (42 mg, 0.129 mmol). The mixture was degassed under vacuum, purged with argon several times, heated to 100 °C and stirred for 2 hours. The mixture was concentrated under reduced pressure, and the residue was purified by silica gel chromatography (dichloromethane/10% methanol in dichloromethane = 0-27/73) to obtain the product as a yellow solid (compound 518c, 17.3 mg, yield 49.3%). LCMS [M+1]⁺ = 540.

### Step 2: Compound 518

### (S)-2,2-Difluoro-N-(4-(2-((1-(piperidin-4-yl)-1H-pyrazol-4-yl)amino)pyrimidin-4-yl)phenyl)cyclopropane-1-carboxamide

4 M hydrogen chloride solution in 1,4-dioxane (0.07 mL) was added to a solution of compound 518c (17.3 mg, 0.028 mmol) in methanol (0.5 mL) and dichloromethane (2 mL). The mixture was stirred at room temperature overnight. The reaction mixture was concentrated under reduced pressure, and the residue was purified by preparative HPLC (formic acid) to obtain the product as a white solid (compound 518, TDM-181118, 13.5 mg, yield 33%). LCMS [M+1]⁺ = 440.

¹H NMR (400 MHz, DMSO) δ 10.76 (s, 1H), 9.47 (s, 1H), 8.45 (d, J = 5.1 Hz, 1H), 8.35 (s, 1H), 8.13 (d, J = 8.7 Hz, 2H), 7.98 (s, 1H), 7.77 (d, J = 8.5 Hz, 2H), 7.62 (s, 1H), 7.24 (d, J = 5.2 Hz, 1H), 4.33 (s, 1H), 3.21 (d, J = 12.5 Hz, 2H), 2.93-2.75 (m, 3H), 2.13-1.87 (m, 6H).

N-(3-((5-chloro-2-((4-(4-methylpiperazin-1-yl)phenyl)amino)pyrimidin-4-yl)oxyphenyl)-2,2-difluorocyclopropane-1-carboxamide (TDM-181120) was synthesized by a similar method to Example 1, as a white solid (19.5 mg, yield 13.7%).

| **TDM NO.** | **Structure** | **LCMS** [M+1]⁺ | **¹H-NMR** |
|---|---|---|---|
| TDM-181120 | 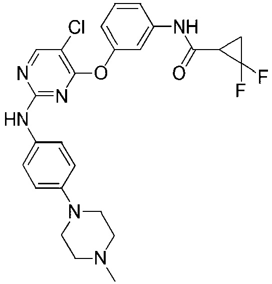 | 515.2 | ¹H NMR (400 MHz, DMSO) δ 10.64 (s, 1H), 9.50 (s, 1H), 8.40 (s, 1H), 8.17 (s, 1H), 7.57-7.49 (m, 2H), 7.44 (t, J = 8.0 Hz, 1H), 7.21 (s, 2H), 7.05-6.94 (m, 1H), 6.65 (s, 2H), 3.02-.94 (m, 4H), 2.86-2.76 (m, 1H), 2.47- 2.40 (m, 4H), 2.22 (s, 3H), 2.07-1.91 (m, 2H). |

### Example 2 General Method for Synthesizing Compound 527 (TDM-181127)

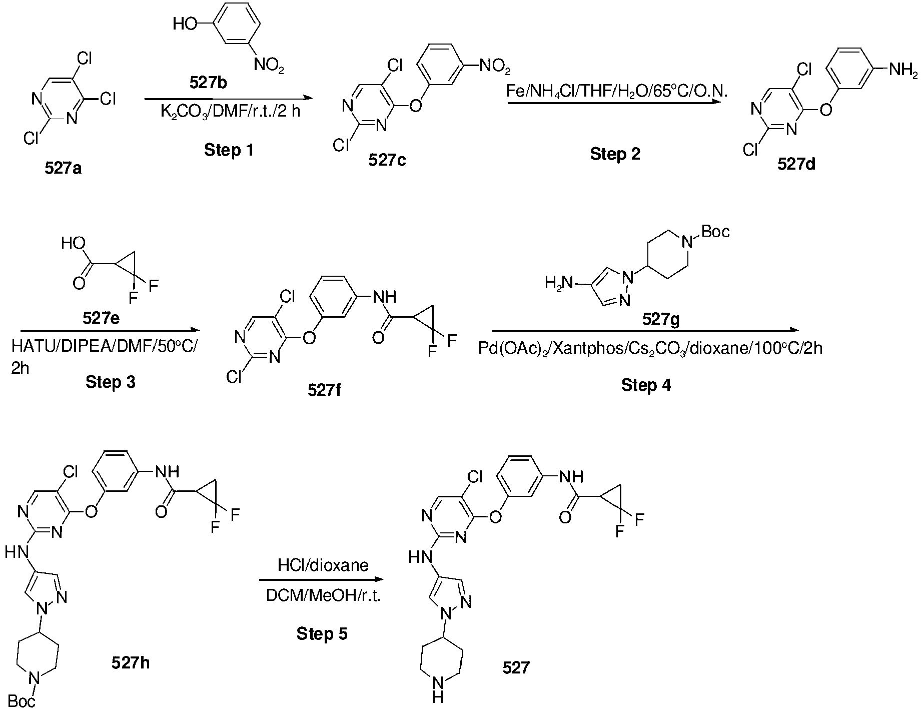

### Step 1: Compound 527c

### 2,5-Dichloro-4-(3-nitrophenoxy)pyrimidine

Potassium carbonate (904 mg, 6.542 mmol) was added to a solution of compound 527a (1000 mg, 5.452 mmol) and compound 527b (758 mg, 5.452 mmol) in N,N-dimethylformamide (40 mL). The mixture was stirred at room temperature for 2 hours. The mixture was then added into water (250 mL) and extracted with ethyl acetate (80 mL × 3). The organic layers were combined, washed with brine, dried over sodium sulfate. The filtrate was concentrated under reduced pressure, and the residue was slurried with dichloromethane and methanol, then filtered to obtain the product as a white solid (compound 527c, 1.34 g, yield 85.9%). LCMS [M+1]⁺ = 286.

### Step 2: Compound 527d

### 3-((2,5-Dichloropyrimidin-4-yl)oxy)aniline

Ammonium chloride (145 mg, 2.622 mmol) and iron powder (148 mg, 2.622 mmol) were added to a mixed solution of compound 527c (150 mg, 0.524 mmol) in tetrahydrofuran (10 mL) and water (10 mL). The mixture was heated to 65 °C and stirred overnight. The mixture was filtered, and the filtrate was concentrated under reduced pressure to remove tetrahydrofuran. The residue was neutralized with aqueous sodium bicarbonate solution and extracted with ethyl acetate (30 mL × 3). The organic layers were combined, washed with brine, dried over sodium sulfate. The filtrate was concentrated under reduced pressure, and the residue was purified by silica gel chromatography (dichloromethane/10% methanol in dichloromethane = 0-1/99) to obtain the product as a white solid (compound 527d, 80 mg, yield 59.6%). LCMS [M+1]⁺ = 256.

### Step 3: Compound 527f

### N-(3-((2,5-Dichloropyrimidin-4-yl)oxy)phenyl)-2,2-difluorocyclopropane-1-carboxamide

N,N-diisopropylethylamine (89 mg, 0.469 mmol) was added to a solution of compound 527d (80 mg, 0.312 mmol), compound 527e (57 mg, 0.469 mmol) and 2-(7-azabenzotriazol-1-yl)-N,N,N',N'-tetramethyluronium hexafluorophosphate (178 mg, 0.469 mmol) in N,N-dimethylformamide (8 mL). The mixture was heated to 50 °C and stirred for 2 hours. The mixture was added into water (80 mL) and extracted with ethyl acetate (50 mL × 3). The organic layers were combined, washed with brine, dried over sodium sulfate. The filtrate was concentrated under reduced pressure, and the residue was purified by silica gel chromatography (dichloromethane/10% methanol in dichloromethane = 0-1/99) to obtain the product as a yellow solid (compound 527f, 70 mg, yield 62.3%). LCMS [M+1]⁺ = 360.

### Step 4: Compound 527h

### Tert-butyl 4-(4-((5-chloro-4-(3-(2,2-difluorocyclopropane-1-carboxamido)phenoxy)pyrimidin-2-yl)amino)-1H-pyrazol-1-yl)piperidine-1-carboxylate

1,4-Dioxane (7 mL) was added to a mixture of compound 527f (70 mg, 0.194 mmol), compound 527g (62 mg, 0.233 mmol), palladium acetate (8.8 mg, 0.039 mmol), xantphos (45 mg, 0.078 mmol) and cesium carbonate (126 mg, 0.388 mmol). The mixture was degassed under vacuum, purged with argon several times, heated to 100 °C and stirred for 2 hours. The mixture was concentrated under reduced pressure, and the residue was purified by silica gel chromatography (dichloromethane/10% methanol in dichloromethane = 0-30/70) to obtain the product as a yellow solid (compound 527h, 55 mg, yield 48%). LCMS [M+1]⁺ = 590.

### Step 5: Compound 527

### N-(3-((5-Chloro-2-((1-(piperidin-4-yl)-1H-pyrazol-4-yl)amino)pyrimidin-4-yl)oxy)phenyl)-2,2-difluorocyclopropane-1-carboxamide

Hydrogen chloride solution in 1,4-dioxane (0.35 mL) was added to a solution of compound 527h (55 mg, 0.093 mmol) in methanol (1 mL) and dichloromethane (5 mL). The mixture was stirred at room temperature overnight. The mixture was concentrated under reduced pressure, and the residue was purified by preparative HPLC (formic acid) to obtain the product as a white solid (compound 527, TDM-181127, 25 mg, yield 54.9%). LCMS [M+1]⁺ = 490.

¹H NMR (400 MHz, MeOD) δ 8.55 (s, 1H), 8.26 (s, 1H), 7.99 (s, 1H), 7.53 (s, 1H), 7.23 (d, J = 30.0 Hz, 2H), 7.10-6.81 (m, 2H), 4.01 (s, 1H), 3.52-3.40 (m, 2H), 3.15 (dd, J = 20.8, 7.9 Hz, 2H), 2.71 (ddd, J = 13.2, 10.8, 7.8 Hz, 1H), 2.20-1.81 (m, 6H).

### Example 3 General Method for Synthesizing Compound 530 (TDM-181130)

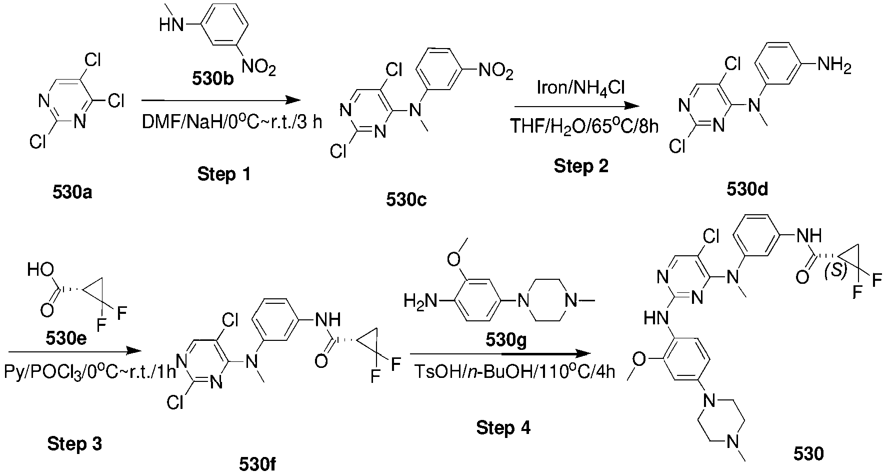

### Step 1: Compound 530c

### 2,5-Dichloro-N-methyl-N-(3-nitrophenyl)pyrimidin-4-amine

Sodium hydride (384 mg, 9.6 mmol) was added to a solution of compound 530b (730 mg, 4.8 mmol) in N,N-dimethylformamide (40 mL) at 0 °C. The mixture was allowed to warm to room temperature naturally and stirred for 1 hour, then compound 530a (1232 mg, 6.72 mmol) was added, and the mixture was stirred at room temperature for 2 hours until the reaction was complete. Workup: The reaction mixture was added into water, and the aqueous phase was extracted with ethyl acetate (3 × 150 mL) three times. The organic phases were combined, washed with saturated brine, dried over anhydrous sodium sulfate, filtered and concentrated to dryness. The obtained crude product was purified by column chromatography [eluent: EA/PE = 0-10%] to obtain the target compound as a yellow solid (compound 530c, 708.6 mg, yield 49.35%). LCMS [M+1]⁺ = 299, 301.

### Step 2: Compound 530d

### N1-(2,5-Dichloropyrimidin-4-yl)-N1-methylbenzene-1,3-diamine

Iron powder (565.3 mg, 10.12 mmol) and ammonium chloride (541.3 mg, 10.12 mmol) were added to a solution of compound 530c (605.5 mg, 2.02 mmol) in tetrahydrofuran (60 mL) and water (60 mL). The reaction mixture was heated to 65 °C and stirred for 8 hours until the reaction was complete. Workup: The reaction mixture was cooled to room temperature and filtered, the filter cake was washed with ethyl acetate. The filtrate was extracted with ethyl acetate three times. The organic phases were combined, washed with saturated brine, dried over anhydrous sodium sulfate, filtered and concentrated to dryness. The obtained crude product was purified by column chromatography [eluent: (dichloromethane/methanol = 10:1)/DCM = 0-40%] to obtain the target compound as a yellow solid (compound 530d, 603 mg, yield 95%). LCMS [M+1]⁺ = 269, 271.

### Step 3: Compound 530f

### (S)-N-(3-((2,5-Dichloropyrimidin-4-yl)(methyl)amino)phenyl)-2,2-difluorocyclopropane-1-carboxamide

Compound 530e (244 mg, 2 mmol) was added to a solution of compound 530d (537.4 mg, 2 mmol) in pyridine (20 mL). The reaction mixture was cooled to 0 °C, then phosphorus oxychloride (0.28 mL, 3 mmol) was added, and the mixture was stirred at this temperature for 1 hour until the reaction was complete. Workup: The reaction mixture was added into ice water (30 mL), and the aqueous phase was extracted with ethyl acetate (2 × 50 mL) twice. The organic phases were combined, washed with saturated brine, dried over anhydrous sodium sulfate, filtered and concentrated to dryness. The obtained crude product was purified by column chromatography [eluent: EA/PE = 0-40%] to obtain the target compound as a yellow solid (compound 530f, 273.5 mg, yield 36.6%). LCMS [M+1]⁺ = 373, 375.

### Step 4: Compound 530

### (S)-N-(3-((5-Chloro-2-((2-methoxy-4-(4-methylpiperazin-1-yl)phenyl)amino)pyrimidin-4-yl)(methyl)amino)phenyl)-2,2-difluorocyclopropane-1-carboxamide

Compound 530g (31 mg, 0.14 mmol) and p-toluenesulfonic acid (53.3 mg, 0.28 mmol) were added to a solution of compound 530f (52 mg, 0.14 mmol) in n-butanol (5 mL). The reaction mixture was heated to 110 °C and stirred for 4 hours until the reaction was complete. Workup: The reaction mixture was added into water, and the aqueous phase was extracted with ethyl acetate (2 × 150 mL) twice. The organic phases were combined, washed with saturated brine, dried over anhydrous sodium sulfate, filtered and concentrated to dryness. The obtained crude product was purified by preparative chromatography to obtain the target compound as a yellow solid (compound 530, 15.4 mg, yield 19.7%). LCMS [M+1]⁺ = 558.

¹H NMR (400 MHz, DMSO) δ 10.46 (s, 1H), 7.98 (s, 1H), 7.87 (s, 1H), 7.73 (d, J = 8.7 Hz, 1H), 7.40 (dd, J = 8.0, 5.0 Hz, 2H), 7.31 (t, J = 8.0 Hz, 1H), 6.91 (d, J = 9.1 Hz, 1H), 6.63 (d, J = 2.5 Hz, 1H), 6.47 (dd, J = 8.8, 2.5 Hz, 1H), 3.83 (s, 3H), 3.36 (s, 3H), 3.15-3.07 (m, 4H), 2.77 (ddd, J = 13.6, 10.9, 8.1 Hz, 1H), 2.47-2.43 (m, 4H), 2.22 (s, 3H), 2.06-1.90 (m, 2H).

(S)-N-(3-((5-Chloro-2-((4-(4-methylpiperazin-1-yl)phenyl)amino)pyrimidin-4-yl)(methyl)amino)phenyl)-2,2-difluorocyclopropane-1-carboxamide (compound 531, TDM-181131) was synthesized by a similar method to Example 3, as an off-white solid (9.4 mg, yield 10%).

| **TDM NO.** | **Structure** | **LCMS** [M+1]⁺ | **¹H-NMR** |
|---|---|---|---|
| TDM-181131 | 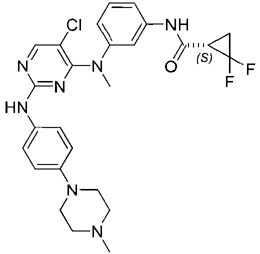 | 529.2 | ¹H NMR (400 MHz, DMSO) δ 10.46 (s, 1H), 9.29 (s, 1H), 8.16 (s, 1H), 8.02 (s, 1H), 7.55 (d, J = 9.1 Hz, 2H), 7.41 (t, J = 4.2 Hz, 2H), 7.31 (t, J = 8.2 Hz, 1H), 6.96-6.82 (m, 3H), 3.41 (s, 3H), 3.08-3.01 (m, 4H), 2.77 (ddd, J = 13.7, 10.9, 8.1 Hz, 1H), 2.47 (s, 4H), 2.23 (s, 3H), 1.98 (dd, J = 13.9, 6.6 Hz, 2H). |

### Example 4 General Method for Synthesizing Compound 532 (TDM-181132)

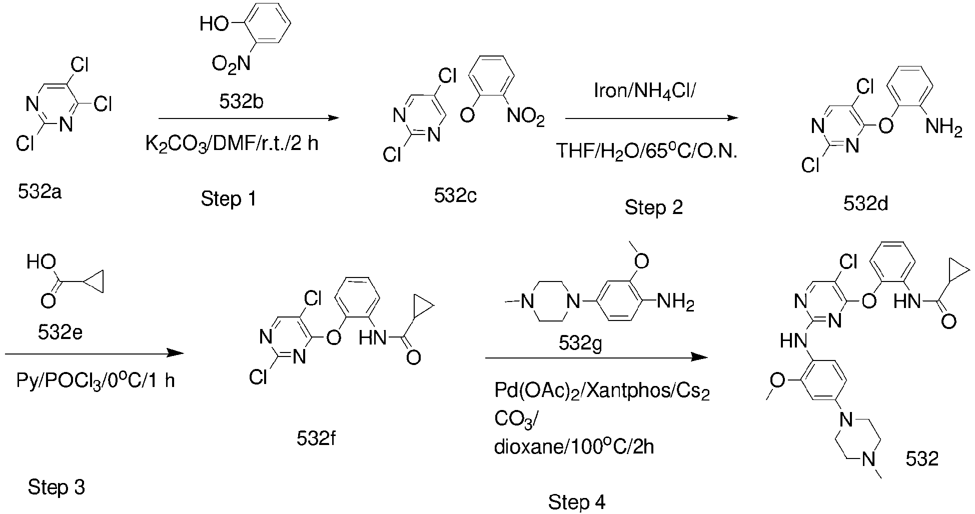

### Step 1: Compound 532c

### 2,5-Dichloro-4-(2-nitrophenoxy)pyrimidine

Compound 532a (550 mg, 3.03 mmol) and potassium carbonate (502 mg, 3.63 mmol) were added to a solution of compound 532b (421 mg, 3.03 mmol) in N,N-dimethylformamide (20 mL). The reaction mixture was stirred at room temperature for 2 hours until the reaction was complete. Workup: The reaction mixture was added into water, and the aqueous phase was extracted with ethyl acetate (3 × 50 mL). The organic phases were combined, washed with saturated brine, dried over anhydrous sodium sulfate, filtered and concentrated to dryness. The obtained crude product was purified by column chromatography [eluent: PE/EA = 0-15%] to obtain the target compound as a white solid (Compound 532c, 800 mg, yield 92.4%). LCMS [M+1]⁺ = 286, 288.

### Step 2: Compound 532d

### 2-((2,5-Dichloropyrimidin-4-yl)oxy)aniline

Iron powder (1.51 g, 27 mmol) and ammonium chloride (1.44 g, 27 mmol) were added to a solution of compound 532c (1.54 g, 5.4 mmol) in tetrahydrofuran (150 mL) and water (150 mL). The reaction mixture was heated to 65 °C and stirred for 3 hours until the reaction was complete. Workup: The reaction mixture was cooled to room temperature and filtered. The filtrate was concentrated to dryness, and water and ethyl acetate (20 mL) were added for extraction three times. The organic phases were combined, washed with saturated brine, dried over anhydrous sodium sulfate, filtered and concentrated to dryness. The obtained crude product was purified by column chromatography [eluent: (dichloromethane/methanol = 10:1)/DCM = 0-40%] to obtain the target compound as a yellow solid (compound 532d, 476 mg, yield 34%). LCMS [M+1]⁺ = 256, 258.

### Step 3: Compound 532f

### N-(2-((2,5-Dichloropyrimidin-4-yl)oxy)phenyl)cyclopropanecarboxamide

Compound 532e (194.5 mg, 1.59 mmol) was added to a solution of compound 532d (408 mg, 1.59 mmol) in pyridine (25 mL). The reaction mixture was cooled to 0 °C, then phosphorus oxychloride (0.22 mL, 2.39 mmol) was added, and the mixture was stirred at this temperature for 1 hour until the reaction was complete. Workup: The reaction mixture was added into ice water (30 mL), and the aqueous phase was extracted with ethyl acetate (2 × 50 mL) twice. The organic phases were combined, washed with saturated brine, dried over anhydrous sodium sulfate, filtered and concentrated to dryness. The obtained crude product was purified by column chromatography [eluent: EA/PE = 0-40%] to obtain the target compound as a yellow solid (compound 532f, 396.8 mg, yield 59%). LCMS [M+1]⁺ = 325.

### Step 4: Compound 532

### N-(2-((5-Chloro-2-((2-methoxy-4-(4-methylpiperazin-1-yl)phenyl)amino)pyrimidin-4-yl)oxy)phenyl)cyclopropanecarboxamide

Compound 532f (30 mg, 0.12 mmol), compound 532g (30.5 mg, 0.14 mmol), palladium acetate (5.16 mg, 0.02 mmol), 4,5-bis(diphenylphosphino)-9,9-dimethylxanthene (26.6 mg, 0.05 mmol), cesium carbonate (75 mg, 0.23 mmol) and 1,4-dioxane (6 mL) were added to a 100 mL three-necked flask. The reaction mixture was purged with argon several times, heated to 100 °C and stirred for 2 hours until the reaction was complete. Workup: The reaction mixture was added into ice water (30 mL), and the aqueous phase was extracted with ethyl acetate (2 × 50 mL) twice. The organic phases were combined, washed with saturated brine, dried over anhydrous sodium sulfate, filtered and concentrated to dryness. The obtained crude product was purified by preparative chromatography to obtain the target compound as a yellow solid (compound 532, 14.3 mg, yield 19.7%). LCMS [M+1]⁺ = 509.

¹H NMR (400 MHz, DMSO) δ 8.30 (s, 1H), 8.07 (s, 1H), 7.72-7.64 (m, 1H), 7.56 (s, 2H), 7.23 (d, J = 5.9 Hz, 3H), 6.58 (d, J = 2.2 Hz, 1H), 6.32 (d, J = 8.4 Hz, 1H), 3.77 (s, 3H), 3.08 (s, 4H), 2.45 (s, 4H), 2.22 (s, 3H), 1.76 (d, J = 5.0 Hz, 1H), 1.00-0.86 (m, 4H).

### Example 5 General Method for Synthesizing Compound 560 (TDM-181160)

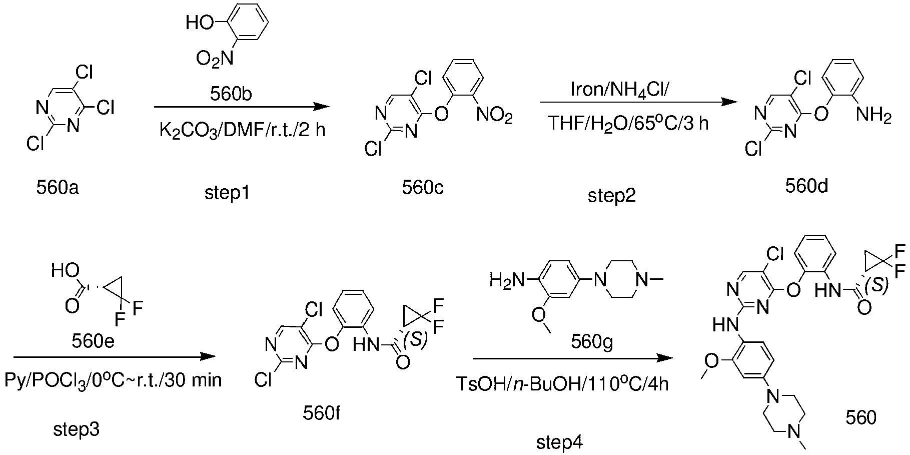

### Step 1: Compound 560c

### 2,5-Dichloro-4-(2-nitrophenoxy)pyrimidine

Compound 560a (550 mg, 3.03 mmol) and potassium carbonate (502 mg, 3.63 mmol) were added to a solution of compound 560b (421 mg, 3.03 mmol) in N,N-dimethylformamide (20 mL). The reaction mixture was stirred at room temperature for 2 hours until the reaction was complete. Workup: The reaction mixture was added into water, and ethyl acetate (3 × 50 mL) was added for layer separation and extraction three times. The organic phases were combined, washed with saturated brine, dried over anhydrous sodium sulfate, filtered and concentrated to dryness. The obtained crude product was purified by column chromatography [eluent: PE/EA = 0-15%] to obtain the target compound as a white solid (compound 560c, 800 mg, yield 92.4%). LCMS [M+1]⁺ = 286, 288.

### Step 2: Compound 560d

### 2-((2,5-Dichloropyrimidin-4-yl)oxy)aniline

Iron powder (1.51 g, 27 mmol) and ammonium chloride (1.44 g, 27 mmol) were added to a solution of compound 560c (1.54 g, 5.4 mmol) in tetrahydrofuran (150 mL) and water (150 mL). The reaction mixture was heated to 65 °C and stirred for 3 hours until the reaction was complete. Workup: The reaction mixture was cooled to room temperature and filtered. The filter cake was washed with ethyl acetate, and the filtrate was extracted with ethyl acetate three times. The organic phases were combined, washed with saturated brine, dried over anhydrous sodium sulfate, filtered and concentrated to dryness. The obtained crude product was purified by column chromatography [eluent: PE/EA = 0-50%] to obtain the target compound as a yellow solid (compound 560d, 476 mg, yield 34%). LCMS [M+1]⁺ = 256, 258.

### Step 3: Compound 560f

### (S)-N-(2-((2,5-Dichloropyrimidin-4-yl)oxy)phenyl)-2,2-difluorocyclopropane-1-carboxamide

Compound 560e (194.5 mg, 1.59 mmol) was added to a solution of compound 560d (408 mg, 1.59 mmol) in pyridine (25 mL). The reaction mixture was cooled to 0 °C, then phosphorus oxychloride (0.22 mL, 2.39 mmol) was added, and the mixture was stirred at this temperature for 30 minutes until the reaction was complete. Workup: The reaction mixture was added into ice water (30 mL), and the aqueous phase was extracted with ethyl acetate (2 × 50 mL) twice. The organic phases were combined, washed with saturated brine, dried over anhydrous sodium sulfate, filtered and concentrated to dryness. The obtained crude product was purified by column chromatography [eluent: PE/EA = 0-20%] to obtain the target compound as a yellow solid (Compound 560f, 396.8 mg, yield 59%). LCMS [M+1]⁺ = 360, 362.

### Step 4: Compound 560

### (S)-N-(2-((5-Chloro-2-((2-methoxy-4-(4-methylpiperazin-1-yl)phenyl)amino)pyrimidin-4-yl)oxy)phenyl)-2,2-difluorocyclopropane-1-carboxamide

Compound 560g (208 mg, 0.94 mmol) and p-toluenesulfonic acid monohydrate (357 mg, 1.88 mmol) were added to a solution of compound 560f (339 mg, 0.94 mmol) in n-butanol (24 mL). The reaction mixture was heated to 110 °C and stirred for 4 hours until the reaction was complete. Workup: The reaction mixture was added into water, and the aqueous phase was extracted with ethyl acetate (2 × 150 mL) twice. The organic phases were combined, washed with saturated brine, dried over anhydrous sodium sulfate, filtered and concentrated to dryness. The obtained crude product was purified by column chromatography [eluent: (dichloromethane/methanol = 10:1)/DCM = 0-50%], then further purified by preparative chromatography to obtain the target compound as a white solid (compound 560, 12.7 mg, yield 17%). LCMS [M+1]⁺ = 545.2.

¹H NMR (400 MHz, DMSO) δ 8.38 (s, 1H), 8.06 (s, 1H), 7.70 (dd, J = 7.0, 3.0 Hz, 1H), 7.55 (d, J = 12.4 Hz, 2H), 7.26 (dd, J = 6.3, 3.7 Hz, 3H), 6.58 (d, J = 2.5 Hz, 1H), 6.31 (d, J = 8.7 Hz, 1H), 3.77 (s, 3H), 3.11-3.07 (m, 4H), 2.99-2.89 (m, 1H), 2.49-2.44 (m, 4H), 2.25 (s, 3H), 2.13 (dt, J = 9.4, 5.8 Hz, 1H), 2.02 (td, J = 14.1, 7.9 Hz, 1H).

### Example 6 General Method for Synthesizing Compound 568 (TDM-181168)

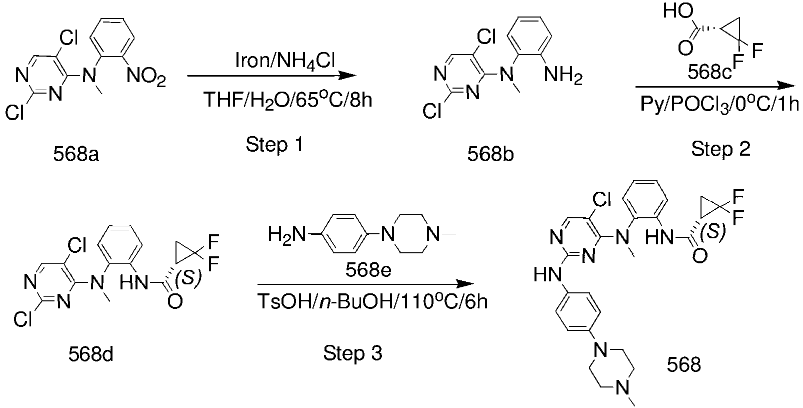

### Step 1: Compound 568b

### N1-(2,5-Dichloropyrimidin-4-yl)-N1-methylbenzene-1,2-diamine

Iron powder (28 mg, 0.502 mmol) and ammonium chloride (26.8 mg, 0.502 mmol) were added to a solution of compound 568a (243 mg, 0.813 mmol) in tetrahydrofuran (2.5 mL) and water (2.5 mL). The mixture was heated to 65 °C and stirred. The mixture was filtered, and the filtrate was concentrated under reduced pressure to remove part of the tetrahydrofuran. The residue was neutralized with aqueous sodium carbonate solution and extracted with ethyl acetate (40 mL × 3). The organic layers were combined, washed with brine, dried over sodium sulfate. The filtrate was concentrated under reduced pressure, and the residue was purified by silica gel chromatography (100% dichloromethane) to obtain the product as a yellow solid (compound 568b, 112.3 mg, yield 45.7%). LCMS [M+1]⁺ = 269, 271.

### Step 2: Compound 568d

### (S)-N-(2-((2,5-Dichloropyrimidin-4-yl)(methyl)amino)phenyl)-2,2-difluorocyclopropane-1-carboxamide

Phosphorus oxychloride (96 mg, 0.626 mmol) was added to a solution of compound 568b (112.3 mg, 0.417 mmol) and compound 568c (61 mg, 0.501 mmol) in pyridine (5 mL) at 0 °C under argon atmosphere. The mixture was stirred at room temperature for 1 hour. The mixture was concentrated under reduced pressure, and the residue was purified by silica gel chromatography (100% dichloromethane) to obtain the product as a yellow solid (Compound 568d, 141 mg, yield 90.6%). LCMS [M+1]⁺ = 373, 375.

### Step 3: Compound 568

### (S)-N-(2-((5-Chloro-2-((4-(4-methylpiperazin-1-yl)phenyl)amino)pyrimidin-4-yl)(methyl)amino)phenyl)-2,2-difluorocyclopropane-1-carboxamide

P-toluenesulfonic acid monohydrate (71.5 mg, 0.376 mmol) was added to a solution of compound 568d (70 mg, 0.188 mmol) and compound 568e (53.8 mg, 0.288 mmol) in n-butanol (5 mL). The mixture was heated to 110 °C and stirred for 6 hours. The mixture was added into water (50 mL) and extracted with ethyl acetate (25 mL × 3). The organic layers were combined, washed with brine. The filtrate was concentrated under reduced pressure, and the residue was purified by preparative HPLC (formic acid) to obtain the product as a white solid (compound 568, TDM-181168, 73.9 mg, yield 58%). LCMS [M+1]⁺ = 528.

¹H NMR (400 MHz, DMSO) δ 9.86 (s, 1H), 9.23 (s, 1H), 8.16 (s, 1H), 8.05 (d, J = 8.2 Hz, 1H), 7.93 (s, 1H), 7.59 (d, J = 9.0 Hz, 2H), 7.32-7.23 (m, 1H), 7.09 (d, J = 4.1 Hz, 2H), 6.89 (s, 2H), 3.23 (s, 3H), 3.20-3.11 (m, 1H), 3.09-3.02 (m, 4H), 2.49-2.43 (m, 4H), 2.23 (s, 3H), 2.09-1.87 (m, 2H).

### Test Example: Enzyme Activity Inhibition Test of NUAK1/NUAK2 Kinase Inhibitors

The inhibitory activity of the pyrimidinamine small molecules involved in the present application against NUAK1 and NUAK2 kinases was determined using a kinase activity assay based on the P81 filter paper binding hot-spot technique (Nat Biotechnol. 2011, 29:1039). A brief description is as follows:

The assay buffer system contained 20 mM Hepes (pH 7.5), 10 mM MgCl₂, 1 mM EGTA, 0.01% Brij35 (L23 polyoxyethylene lauryl ether), 0.02 mg/mL BSA (bovine serum albumin), 0.1 mM Na₃VO₄, 2 mM DTT, and 1% DMSO.

### Test Steps:

1. A 20 µM substrate solution was prepared with freshly prepared test buffer and CHKtide peptide fragments (Sanchez Y. Science. 1997, 277: 1497-1501) were used as a substrate.
2. Kinases were added and mixed gently (a final concentration of NUAK1 was 10 nM; and a final concentration of NUAK2 was 50 nM).
3. Test compounds dissolved in 100% DMSO were each added to the above kinase reaction mixture solution via Acoustic Droplet Ejection (Echo550; nanoliter) and the mixture was incubated for 5 minutes at room temperature.
4. ³³P-labeled ATP (a ratio of unlabeled ATP to labeled ATP was 25:1 or 2.5:1) was added.
5. The mixture was incubated for 2 hours at room temperature.
6. Kinase activity was tested using the P81 filter paper combined with hotspot technology.

The IC₅₀ values of the test compounds against NUAK1/NUAK2 were calculated according to the above test. For specific results, see Table 1.

The IC₅₀ values were calculated by using a formula derived from sigmoidal dose-response curve (variable slope):

Y = Bottom + (Top-Bottom)/(1+10^((LogEC50-X)*Slope, where X is the logarithm of compound concentration, Y is the response (inhibition rate of kinase activity), and Y rises from bottom to top along the sigmoidal curve as the concentration increases.

**Table 1: IC₅₀ (nM) of Test Compounds of the Present Application against NUAK1/NUAK2**

| Test compound | NUAK1 | NUAK2 |
|---|---|---|
| TDM-181120 | 21 | 311 |
| TDM-181127 | 161 | 352 |
| TDM-181131 | 81 | 635 |
| TDM-181132 | 24 | 37 |
| TDM-181160 | 4 | 13 |

As can be seen from the results in Table 1, the pyrimidinamine-based compounds of the present application exhibit excellent inhibitory activity against both NUAK1 and NUAK2, and are dual-target small-molecule kinase inhibitors. The IC₅₀ values of the compounds against NUAK1/NUAK2 can reach several nM to several tens of nM. Therefore, the above experiments demonstrate that the pyrimidinamine-based compounds of the present application can be used as NUAK1/NUAK2 inhibitors.

The preferred embodiments of the present disclosure have been described in detail above. However, the present disclosure is not limited to the specific details in the above embodiments. Within the scope of the technical concept of the present disclosure, various simple modifications can be made to the technical solutions of the present disclosure, and all such simple modifications fall within the scope of protection of the present disclosure.

In addition, it should be noted that the various specific technical features described in the above specific embodiments may be combined in any suitable manner without contradiction. To avoid unnecessary repetition, the present disclosure does not separately describe every possible combination.

Moreover, any combination of the various embodiments of the present disclosure may be made, provided that such combination does not depart from the spirit of the present disclosure, and such combinations shall also be regarded as the content disclosed in the present disclosure.

## Claims

1. A pyrimidinamine-based compound, wherein the pyrimidinamine-based compound is a compound represented by Formula I, or a stereoisomer, a tautomer, an isotopic derivative, a hydrate, a solvate, a prodrug, or a pharmaceutically acceptable salt thereof, 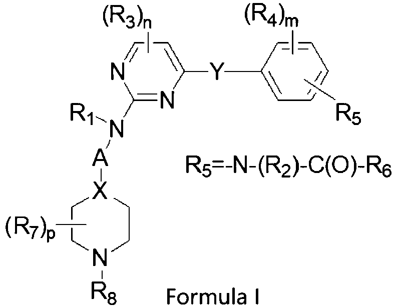
wherein A is 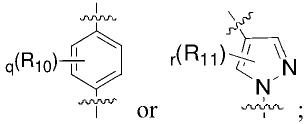
X is N or CH, and Y is O or NR₉;
R₁, R₂, R₈, and R₉ are each independently H or an optionally substituted C1-C8 alkyl group; R₃, R₄, R₇, R₁₀, and R₁₁ are each independently halogen, an amino group, a hydroxyl group, a nitro group, a cyano group, a sulfhydryl group, an optionally substituted C1-C8 alkyl group, an optionally substituted C1-C8 alkoxy group, an optionally substituted C1-C8 alkylthio group, or an optionally substituted C1-C8 alkylamino group;
R₆ is an optionally substituted 3- to 10-membered cycloalkyl group or an optionally substituted 3- to 10-membered heterocycloalkyl group, wherein the substituent on the cycloalkyl group or heterocycloalkyl group is one or more selected from the group consisting of halogen, an amino group, a hydroxyl group, a nitro group, a cyano group, a sulfhydryl group, an optionally substituted C1-C8 alkyl group, an optionally substituted C1-C8 alkoxy group, an optionally substituted C1-C8 alkylthio group, and an optionally substituted C1-C8 alkylamino group;
the substituent on the C1-C8 alkyl group is one or more selected from the group consisting of halogen, an amino group, a hydroxyl group, a nitro group, a cyano group, and a sulfhydryl group; and
n and r are each 0, 1, or 2; and m, p, and q are each 0, 1, 2, 3, or 4.

2. The compound according to claim 1, wherein Y is O or NH.

3. The compound according to any one of claims 1 to 2, wherein R₆ is an optionally substituted 3- to 10-membered cycloalkyl group.

4. The compound according to claim 3, wherein R₆ is an optionally substituted 3- to 6-membered cycloalkyl group.

5. The compound according to claim 4, wherein R₆ is a 3- to 6-membered cycloalkyl group substituted with halogen.

6. The compound according to claim 5, wherein R₆ is a 3- to 6-membered cycloalkyl group substituted with fluorine.

7. The compound according to claim 4, wherein R₆

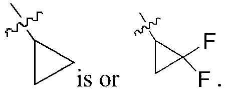

8. The compound according to claim 7, wherein R₅ is

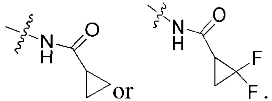

9. The compound according to claim 8, wherein R₅ is

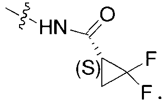

10. The compound according to any one of claims 1 to 9, wherein R₅ is substituted at the ortho-or meta-position to Y.

11. The compound according to any one of claims 1 to 10, wherein the compound represented by Formula I is represented by Formula I-1:

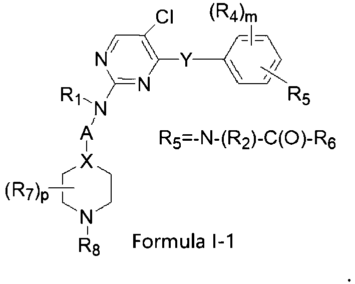

12. The compound according to claim 11, wherein X is N, and R₈ is H or a methyl group.

13. The compound according to claim 12, wherein the compound represented by Formula I is represented by Formula I-2 or Formula I-3:

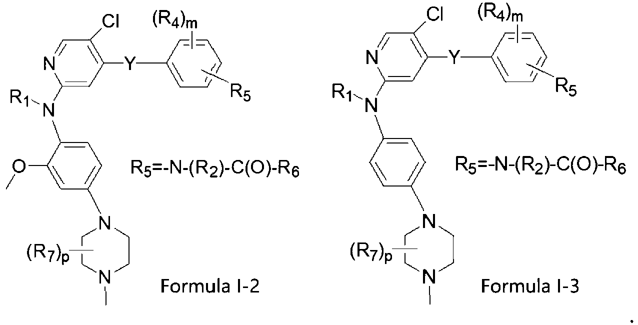

14. The compound according to any one of claims 1 to 13, wherein the compound represented by Formula I is one of the following compounds:

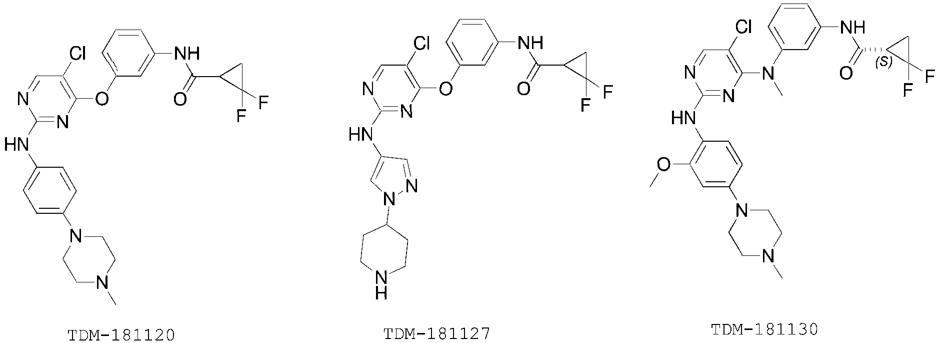

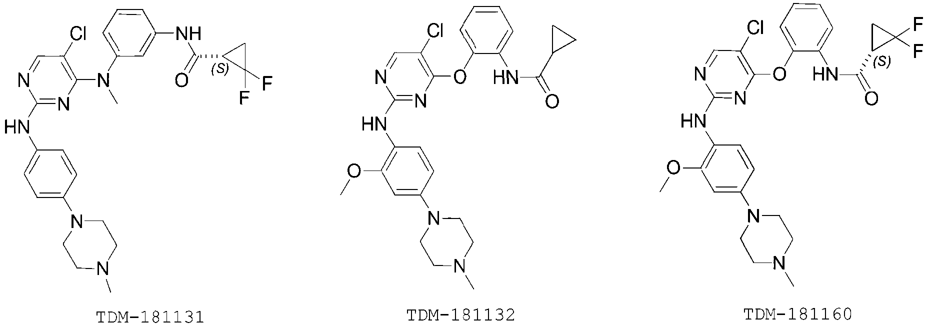

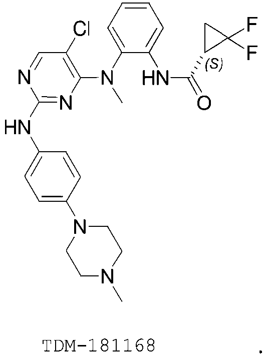

15. The compound according to any one of claims 1 to 14, wherein the pharmaceutically acceptable salt is a formate salt.

16. The compound according to any one of claims 1 to 15, wherein the isotopic derivative is a deuterated substance.

17. A method for preparing the compound according to any one of claims 1 to 16, comprising:
preparing the compound of Formula I from a compound of Formula II and a compound of Formula III; or
preparing a compound of Formula V from a compound of Formula IV and a compound of Formula II, and then preparing the compound of Formula I from the compound of Formula V;
optionally, the preparation process comprises necessary protection and deprotection steps, 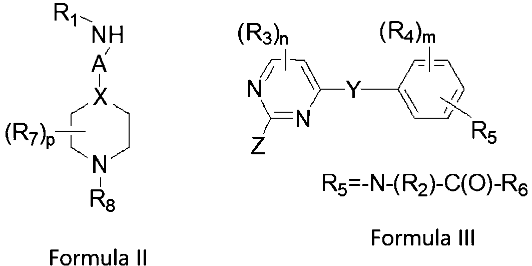 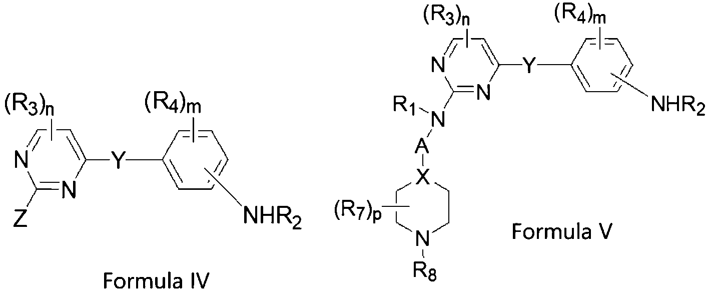
wherein Z is a leaving group, preferably Z is halogen, more preferably Z is Cl, and other groups are as defined in any one of claims 1 to 16.

18. A pharmaceutical composition comprising the compound according to any one of claims 1 to 16 as an active ingredient.

19. The pharmaceutical composition according to claim 18, comprising a pharmaceutically acceptable carrier or excipient.

20. Use of the compound according to any one of claims 1 to 16 in preparation of a NUAK1 or NUAK2 inhibitor.

21. Use of the compound according to any one of claims 1 to 16 in preparation of a medicament, wherein the compound is used for the prevention or treatment of the following diseases by inhibiting NUAK1 or NUAK2: neuropsychiatric diseases, metabolic diseases, tumors, visceral fibrotic diseases, or skin fibrotic diseases.

22. The use according to claim 21, wherein the disease is Parkinson's disease, Alzheimer's disease, diabetes, hyperlipidemia, obesity, liver cancer, leukemia, lymphoma, tumor metastasis, liver cirrhosis, renal fibrosis, pulmonary fibrosis, post-myocarditis sequelae, scleroderma, keloids, or hypertrophic scar; or the compound is used solely for alleviating a traumatic or postoperative scar.
